# NOUVEAU FASCICULE DE BREVET EUROPEEN

(11) **EP 2 145 051 B2**
(45) Date de publication et mention de la décision concernant l'opposition: **22.01.2025**
(45) Mention de la délivrance du brevet: 12.02.2020
(21) Numéro de dépôt: 08805500.9
(22) Date de dépôt: 23.04.2008
(51) Int. Cl.: E03B 1/02, C02F 1/00, G01N 33/18, E03B 7/02

(54) **PROCEDE ET INSTALLATION DE CONTROLE EN TEMPS REEL DE LA QUALITE DE L'EAU D'UN RESEAU DE DISTRIBUTION**
VERFAHREN UND ANLAGE ZUR ECHTZEITÜBERPRÜFUNG DER WASSERQUALITÄT IN EINEM LIEFERNETZWERK
METHOD AND INSTALLATION FOR REAL-TIME INSPECTION OF THE QUALITY OF WATER IN A DELIVERY NETWORK

(30) Priorité: 04.05.2007 FR 0703249
(43) Date de publication de la demande: 20.01.2010
(73) Titulaire: Suez International, 92040 Paris La Défense Cedex (FR)
(72) Inventeur: PHAM, Hao-Nhiên, F-75013 Paris (FR); LAINE, Jean-Michel, F-78920 Ecquevilly (FR); KORA, Roland, F-78160 Marly Le Roi (FR)
(74) Mandataire: Lavoix
(86) Numéro de dépôt international: PCT/FR2008/000581
(87) Numéro de publication internationale: WO 2008/148952

(56) Documents cités:
- US-A- 4 562 552
- US-A1- 2002 130 069
- US-A1- 2004 006 513
- US-A1- 2005 279 169
- US-A1- 2007 090 059
- US-B1- 6 245 224
- RICHARD JARRETT, GEOFF ROBINSON: "On-line Monitoring of water distribution systems: date processing and anomaly detection", 8TH ANNUAL WATER DISTRIBUTION SYSTEMS ANALYSIS SYMPOSIUM, 27 August 2006 (2006-08-27) - 30 August 2006 (2006-08-30), pages 1 - 14
- SOUBESTE DAVY: "Pollutec 2006 : Coronis Systems lance un afficheur digital pour une meilleure gestion des energies", CATEGORYNET.COM, 13 November 2006 (2006-11-13)
- ANONYMOUS: "Un afficheur digital pour une meilleure gestion des énergies", 14 November 2006 (2006-11-14), pages 1 - 5, Retrieved from the Internet <URL:https://www.industrie-techno.com/article/un-afficheur-digital-pour-une-meilleure-gestion-des-energies.5621>
- "Coronis Systems presente Wavelook Ce moniteur leger et sans fil permet aux utilisateurs de mieux gerer leur consommation d’eau et d’energie”, Maintenance facilitee. 2006
- SAUNIER, B.M. et al., 'Evolution de la qualite de I’eau dans les reseaux de distribution d'eau potable : diagnostic et modelisation", Journees d'mformation Eaux, 27 septembre 1990
- Clark, Robert M. et al., "Modeling Chlorine Residuals in Drinking-Water Distribution Systems" Journal of Environmental Engineering, juillet 1994
- Walski, Thomas M. el al., "Water Distribution Modeling" University of Dayton: Civil and Environmental Engineering and Engineering Mechanics Facultv Publications. 2001

## Description

L'invention est relative à un procédé de contrôle de la qualité de l'eau d'un réseau de distribution d'eau potable comportant, d'une part, sur des branchements pour consommateurs, des compteurs de consommation et, d'autre part, des analyseurs en ligne répartis en des points de surveillance sur le réseau pour la mesure d'au moins un paramètre de qualité de l'eau.

Des modèles hydrauliques sont utilisés généralement pour donner une évaluation de l'état du réseau de distribution en ce qui concerne débits et pressions. Par ailleurs un système de télégestion donne l'état d'ouverture ou de fermeture de vannes sur le réseau.

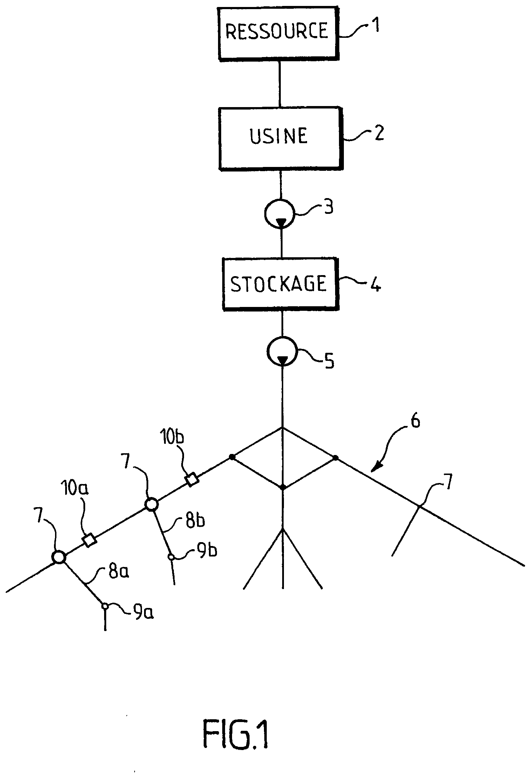

Comme illustré sur la figure 1 des dessins annexés, une distribution d'eau potable fait appel à une ressource 1 qui, par exemple, peut être un cours d'eau ou une source captée. La ressource 1 alimente en eau une usine de traitement 2 dont la sortie est reliée à une installation de pompage 3 qui envoie l'eau à un ou plusieurs réservoirs de stockage 4. L'eau de la ressource est traitée dans l'usine 2 où les éléments indésirables à la qualité de l'eau potable sont éliminés. La sortie d'un réservoir de stockage 4 est reliée soit à des moyens de pompage 5 qui délivrent l'eau potable, soit directement en gravitaire à un réseau maillé de distribution 6 comportant des points 7 de branchement de conduites telles que 8a, 8b... pour alimenter des consommateurs. Chaque conduite 8a, 8b... est équipée d'un compteur de consommation respectif 9a, 9b.

La qualité de l'eau fournie au consommateur, en aval des compteurs tels que 9a, 9b,... est un souci majeur pour les entreprises de distribution d'eau.

Des paramètres de qualité de l'eau, tels que la teneur en chlore, ou la turbidité, évoluent entre la sortie de l'usine de traitement 2 et le compteur de consommation 9a, 9b,... en fonction notamment de la géométrie du réseau, de la distance parcourue par l'eau, du volume et du débit de consommation. Des analyseurs en ligne tels que 10a, 10b,... sont parfois en certains points de contrôle du réseau pour la mesure d'au moins un paramètre de qualité de l'eau, en particulier la teneur en chlore.

La maîtrise de la qualité de l'usine de traitement 2 jusqu'au réservoir 4 est aisée. Par ailleurs la ressource 1, l'usine de traitement 2, le pompage 3, le ou les réservoirs de stockage 4, et le pompage 5 étant bien localisés et limités géographiquement, ils peuvent être efficacement surveillés contre toute intrusion non autorisée susceptible d'affecter la qualité de l'eau sortant du réservoir 4.

Par contre, au niveau du réseau maillé 6 enterré, la maîtrise en temps réel de la qualité de l'eau en différents endroits est difficile. Le réseau 6 est largement étendu et il existe de nombreuses possibilités d'altération de la qualité de l'eau. Tout point de connexion au réseau peut être considéré comme un point d'intrusion potentiel pour des éléments pouvant affecter la qualité de l'eau. Il en résulte que la surveillance du réseau maillé 6 des utilisateurs finaux contre des intrusions accidentelles ou malveillantes est un problème délicat.

D'une manière courante, la qualité de l'eau d'un réseau tel que 6 est vérifiée selon des programmes de surveillance légaux avec des analyses de quelques paramètres de qualité, en général à une fréquence quotidienne. Cette fréquence basse n'est pas compatible avec une réponse rapide souhaitée pour une surveillance en temps réel.

L'accroissement de la demande des utilisateurs et les règlements en matière de qualité de l'eau ont conduit les entreprises de distribution d'eau à installer des analyseurs en ligne, spécialement des analyseurs de chlore pour la surveillance de la qualité bactériologique. D'autres paramètres de qualité que le chlore peuvent être pris en compte, notamment la turbidité. Le niveau résiduel de chlore dans un réseau de distribution d'eau montre toujours une variation qui correspond à une évolution normale quotidienne dépendant de la consommation d'eau effective et de l'état hydraulique du réseau. Il en résulte une difficulté réelle à détecter et à établir une situation anormale en terme de niveau de chlore. Il en est de même pour d'autres paramètres de qualité de l'eau. La fixation d'un seuil simple et constant pour un niveau du paramètre de qualité n'est ainsi pas suffisante pour une détection rapide et fiable d'une situation anormale.

US 2004/0006513 A1 concerne un système de surveillance d'un réseau de distribution d'eau pour le protéger contre une contamination biologique ou chimique. Il est prévu de surveiller de paramètres du réseau notamment le débit, la teneur en chlore libre, ainsi que la turbidité et de comparer les mesures à des données historiques. Les mesures sont effectuées au départ de l'usine des eaux ou du château d'eau, ainsi que sur le réseau pour une stratégie de distribution. De telles mesures permettent d'obtenir des indications suffisantes pour piloter une stratégie de distribution, mais sont insuffisantes pour valider une alarme. En particulier, une mesure indiquant un flux inverse ne signifie pas forcément une anormalité ou une intrusion dans le réseau : il peut s'agir d'un simple équilibrage dans un réseau maillé entre deux sources de production (par exemple deux usines, ou deux châteaux d'eau).

L'invention a pour but, surtout, de fournir un procédé de contrôle en temps réel de la qualité de l'eau d'un réseau de distribution qui permet d'améliorer sensiblement l'évaluation de la teneur en un paramètre de qualité de l'eau distribuée aux divers points de consommation. L'invention a également pour but de permettre une détection rapide d'une chute de qualité, en particulier par suite d'une intrusion accidentelle ou malveillante dans le réseau de distribution.

Selon l'invention, un procédé de contrôle de la qualité de l'eau d'un réseau de distribution d'eau potable comportant, d'une part, des compteurs de consommation, et d'autre part des analyseurs en ligne répartis en des points de surveillance sur le réseau pour la mesure d'au moins un paramètre de qualité de l'eau, procédé selon lequel :
- les mesures des analyseurs sont transmises à une unité de calcul,
- l'unité de calcul établit des valeurs estimées de la concentration dans l'eau du paramètre considéré aux différents points de surveillance du réseau,
- et un système de pré-alerte effectue une comparaison entre les valeurs estimées du paramètre de qualité et les valeurs mesurées en différents points du réseau, une alerte étant déclenchée lorsque l'écart entre la valeur mesurée et la valeur estimée dépasse un seuil prédéterminé,
est caractérisé en ce que, pour un contrôle en temps réel,
- des compteurs équipés de dispositifs de télérelevé sont installés sur des branchements pour consommateurs,
- les données de consommation des différents compteurs pour consommateurs sont transmises à l'unité de calcul,
- l'unité de calcul est programmée avec un modèle hydraulique et un modèle cinétique de décroissance du paramètre de qualité considéré,
- et l'unité de calcul met à jour en permanence le modèle hydraulique selon les données de consommation reçues des compteurs pour consommateurs.

En particulier, une consommation anormale en chlore pourrait signifier l'intrusion d'un contaminant biologique.

L'installation de compteurs de consommation à télérelevé permet de transmettre à distance, généralement par voie hertzienne, à un récepteur fixe les consommations relevées par chaque compteur. Le récepteur fixe recueille toutes les données provenant de son environnement. Ces données sont ensuite envoyées à un système centralisé qui peut offrir aux consommateurs des services tels que détection de fuite, détection de compteur bloqué, relevés de consommation plus fréquents.

Avantageusement, une information de flux inverse à partir d'un compteur peut être transmise au système de pré-alerte et les informations groupées sur le flux inverse et la teneur en paramètre de qualité sont combinées pour augmenter le niveau de probabilité de la détection d'une anormalité ou d'une intrusion dans le réseau.

Selon l'invention, une mesure d'un flux inverse sur le compteur d'un consommateur signifie obligatoirement une intrusion sur le réseau, et donc une anormalité et une alarme.

De préférence, les données de consommation des différents compteurs équipés de dispositifs de télérelevé sont transmises à l'unité de calcul au moins une fois par heure.

Le paramètre de qualité de l'eau, pris en compte pour le contrôle du réseau en temps réel, peut être constitué par la teneur de l'eau en chlore.

L'invention concerne également une installation pour la mise en œuvre du procédé défini précédemment, pour un réseau de distribution d'eau potable qui comporte, d'une part, des compteurs de consommation et, d'autre part des analyseurs en ligne répartis en des points de contrôle sur le réseau pour la mesure d'au moins un paramètre de qualité de l'eau, laquelle installation comporte également :
- une unité de calcul à laquelle sont transmises les mesures des analyseurs, cette unité de calcul établissant des valeurs estimées de la concentration dans l'eau du paramètre considéré aux différents points de surveillance du réseau,
- et un système de pré-alerte qui effectue une comparaison entre les valeurs estimées du paramètre de qualité et les valeurs mesurées en différents points du réseau, une alerte étant déclenchée lorsque l'écart entre la valeur mesurée et la valeur estimée dépasse un seuil prédéterminé,
laquelle installation est caractérisée en ce que, pour un contrôle en temps réel,
- ladite installation comporte des compteurs équipés de dispositifs de télérelevé installés sur des branchements pour consommateurs,
- les données de consommation des différents compteurs pour consommateurs sont transmises à l'unité de calcul,
- l'unité de calcul est programmée avec un modèle hydraulique et un modèle cinétique de décroissance du paramètre de qualité considéré,
- et l'unité de calcul met à jour en permanence le modèle hydraulique selon les données de consommation reçues des compteurs pour consommateurs.

Avantageusement, l'installation comporte un système de télérelevé fixe qui établit, à partir de toutes les informations de consommation transmises par les compteurs, une estimation de la consommation instantanée, qui est communiquée par une liaison à l'unité de calcul.

Le système de télérelevé peut être prévu pour signaler un flux inverse, cette information de flux inverse étant transmise par une liaison au système de pré-alerte.

L'installation comporte, de préférence, un système de télésurveillance qui reçoit les informations des analyseurs et qui transmet, après traitement, à l'unité de calcul les informations sur l'état de la pression et du débit dans le réseau, et par au moins une ou plusieurs liaisons les informations sur la concentration dans l'eau du paramètre de qualité.

Avantageusement, des analyseurs mesurent la teneur en chlore de l'eau qui constitue un paramètre de qualité.

L'invention consiste, mises à part les dispositions exposées ci-dessus, en un certain nombre d'autres dispositions dont il sera plus explicitement question ci-après à propos d'un exemple de réalisation décrit avec référence aux dessins annexés, mais qui n'est nullement limitatif. Sur ces dessins :
Fig. 1 est un schéma d'un réseau de distribution d'eau, et
Fig. 2 est un schéma d'une installation équipée d'un dispositif pour la mise en œuvre du procédé de l'invention.

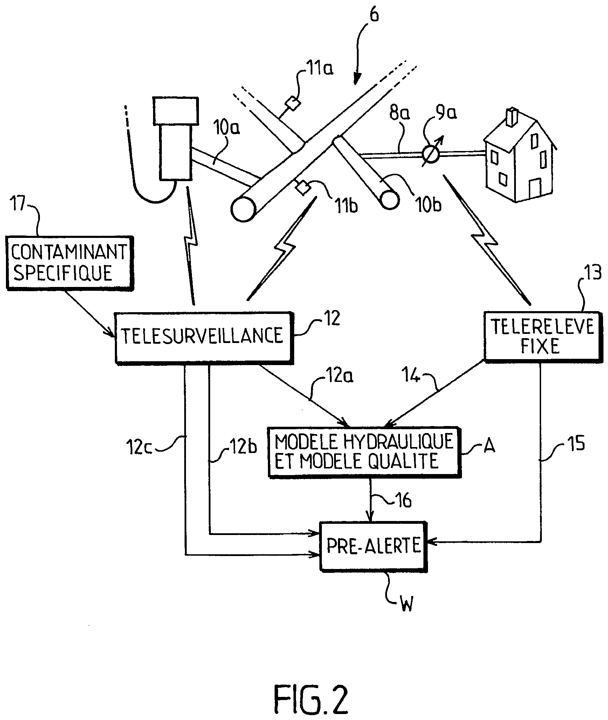

Comme illustré sur Fig. 2, l'installation pour la mise en œuvre du procédé de l'invention comprend un système de télésurveillance 12 auquel sont transmises les informations provenant d'analyseurs tels que 10a... mesurant la teneur de l'eau en un paramètre de qualité, en particulier en chlore, ainsi que les informations de débit et de pression provenant des capteurs 11a, 11b... Les informations sont transmises fréquemment, notamment au moins une fois par heure, par voie hertzienne.

L'installation comprend également un système de télérelève fixe 13 qui reçoit périodiquement, au moins une fois par heure, les informations de consommation des compteurs tels que 9a équipés de télérelevés.

Le système de télérelevé fixe 13 établit, à partir de toutes les informations de consommation transmises par les compteurs, une estimation relativement précise de la consommation instantanée, qui est communiquée selon une liaison 14 à une unité de calcul et de traitement A. Un modèle hydraulique et un modèle qualité sont chargés dans l'unité A.

Le système de télérelevé fixe 13 signale en outre un flux inverse éventuel correspondant à un compteur tel que 9a qui, au lieu d'indiquer une augmentation de consommation, indique une diminution. Un tel flot inverse peut être dû à une intrusion dans le réseau, accidentelle ou malveillante. L'information de flux inverse est transmise, par une liaison 15, du système 13 à un système W de pré-alerte.

Le système de télésurveillance 12 transmet à l'unité de calcul A, par une liaison 12a, les informations sur l'état de la pression et du débit dans le réseau. En outre, le système 12 transmet, par des liaisons 12b, 12c, au système de pré-alerte W les valeurs mesurées pour le ou les paramètres de qualité. Dans l'exemple considéré, la voie 12b transmet les mesures de teneur en chlore aux différents points analysés du réseau et la voie 12c transmet des mesures de turbidité. L'unité A de calcul est programmée avec le modèle hydraulique et un modèle cinétique de décroissance du paramètre de qualité considéré à partir de la sortie de l'usine de traitement jusqu'en un point du réseau.

L'unité de calcul A donne à partir des informations de consommation d'eau, de pression et débit dans le réseau, et à partir des teneurs des paramètres de qualité mesurées en sortie d'usine de traitement 2, une évaluation de la teneur de l'eau en ces paramètres de qualité, notamment teneur en chlore, en différents points du réseau. Cette évaluation est fournie, par une liaison 16, au système de pré-alerte W.

Il est à noter que le chlore est consommé en permanence dans l'eau distribuée du fait de la présence éventuelle de composants biologiques. La connaissance des consommations en eau fournies par les compteurs tels que 9a équipés de télérelevés, permet d'effectuer une prédiction précise et fiable de la teneur de l'eau en chlore, et plus généralement du paramètre de qualité retenu, aux différents points du réseau.

Des informations sur un contaminant spécifique peuvent être fournies à partir d'un bloc 17 au système de télésurveillance 12.

Le système de pré-alerte W compare les valeurs mesurées arrivant par les liaisons 12b et 12c pour les paramètres retenus, notamment pour la teneur en chlore, et les valeurs estimées fournies par la liaison 16 pour les différents points où sont installés des analyseurs. Lorsque la différence entre la valeur mesurée et la valeur estimée attendue dépasse une limite prédéterminée, le système W déclenche une alerte par tout moyen approprié.

Du fait que les valeurs estimées tiennent compte de la décroissance naturelle des paramètres et des consommations réelles, un écart entre valeur estimée attendue et valeur mesurée peut être attribué avec plus de certitude à une intrusion dans le réseau que dans le cas où la décroissance et les consommations ne sont pas prises en compte de manière précise.

L'analyse d'un flux inverse éventuel fourni par la liaison 15 constitue un élément déclenchant supplémentaire de l'alerte.

La teneur en chlore constitue un très bon traceur de qualité de l'eau. Si une intrusion biologique, ou chimique, a lieu dans le réseau, en amont de points de mesure équipés d'analyseurs, elle fait baisser la teneur en chlore au niveau des analyseurs voisins de l'intrusion. La teneur en chlore mesurée deviendra alors sensiblement inférieure à la teneur estimée fournie par calcul sur la liaison 16, et permettra de déclencher une alerte.

Les transmissions d'informations sur l'état du réseau de distribution permettent de déterminer si des vannes sont ouvertes ou fermées.

Les informations groupées sur le flux inverse et sur la teneur en paramètre de qualité, en particulier en chlore, peuvent être combinées pour augmenter le niveau de probabilité de la détection d'un défaut ou d'une intrusion dans le réseau.

Selon l'invention la surveillance s'effectue à trois niveaux :
- Niveau 1 : mesures au départ de l'usine ou du château d'eau : contrôle des dosages de réactifs ;
- Niveau 2 : mesures sur les réseaux avec analyseurs tels que 10a, 10b, et capteurs 11a, 11b, permettant une stratégie de distribution ;
- Niveau 3 : mesures en temps réel sur les compteurs 9a, 9b... des consommateurs, permettant une surveillance de l'eau distribuée et une alerte très rapide.

Le procédé et l'installation selon l'invention permettent de surveiller en temps réel la qualité de l'eau distribuée, et peuvent servir à détecter une intrusion malveillante correspondant à une contamination intentionnelle de l'eau du réseau.

## Revendications

1. Procédé de contrôle de la qualité de l'eau d'un réseau de distribution d'eau potable comportant, d'une part, des compteurs de consommation, et d'autre part des analyseurs en ligne répartis en des points de surveillance sur le réseau pour la mesure d'au moins un paramètre de qualité de l'eau, procédé selon lequel :
- les mesures des analyseurs (10a, 10b...) sont transmises à une unité de calcul (A),
- l'unité de calcul (A) établit des valeurs estimées de la concentration dans l'eau du paramètre considéré aux différents points de surveillance du réseau,
- des valeurs mesurées du paramètre de qualité en différents points du réseau, sont transmises à un système de pré-alerte (W),
- et le système de pré-alerte (W) effectue une comparaison entre les valeurs estimées du paramètre de qualité et les valeurs mesurées en différents points du réseau, une alerte étant déclenchée lorsque l'écart entre la valeur mesurée et la valeur estimée dépasse un seuil prédéterminé, **caractérisé en ce que,** pour un contrôle en temps réel,
- des compteurs (9a, 9b...) équipés de dispositifs de télérelevé sont installés sur des branchements pour consommateurs,
- les données de consommation des différents compteurs (9a, 9b...) pour consommateurs sont transmises à l'unité de calcul (A),
- l'unité de calcul (A) est programmée avec un modèle hydraulique et un modèle cinétique de décroissance du paramètre de qualité considéré,
- et l'unité de calcul (A) met à jour en permanence le modèle hydraulique selon les données de consommation reçues des compteurs (9a, 9b...) pour consommateurs,
- une information de flux inverse est transmise (15) au système de pré-alerte (W) à partir d'un compteur (9a,...) et les informations groupées sur le flux inverse et la teneur en paramètre de qualité sont combinées pour augmenter le niveau de probabilité de la détection d'une anormalité ou d'une intrusion dans le réseau.

2. Procédé selon la revendication 1, **caractérisé en ce que** les données de consommation des différents compteurs (9a, 9b...) équipés de dispositifs de télérelevé sont transmises à l'unité de calcul (A) au moins une fois par heure.

3. Procédé selon l'une des revendications 1 à 2, **caractérisé en ce que** le paramètre de qualité de l'eau, pris en compte pour le contrôle du réseau en temps réel, est constitué par la teneur de l'eau en chlore.

4. Installation pour la mise en oeuvre d'un procédé selon l'une quelconque des revendications précédentes, pour un réseau de distribution d'eau potable, qui comporte, d'une part, des compteurs de consommation et, d'autre part des analyseurs en ligne répartis en des points de contrôle sur le réseau pour la mesure d'au moins un paramètre de qualité de l'eau, laquelle installation comporte également
- une unité de calcul (A) à laquelle sont transmises les mesures des analyseurs (10a, 10b...), cette unité de calcul (A) établissant des valeurs estimées de la concentration dans l'eau du paramètre considéré aux différents points de surveillance du réseau,
- et un système de pré-alerte (W) auquel sont transmises des valeurs mesurées du paramètre de qualité en différents points du réseau qui effectue une comparaison entre les valeurs estimées du paramètre de qualité et les valeurs mesurées en différents points du réseau, une alerte étant déclenchée lorsque l'écart entre la valeur mesurée et la valeur estimée dépasse un seuil prédéterminé,
**caractérisée en ce que,** pour un contrôle en temps réel,
- l'installation comporte des compteurs (9a, 9b...) équipés de dispositifs de télérelevé installés sur des branchements pour consommateurs,
- les données de consommation des différents compteurs (9a, 9b...) pour consommateurs sont transmises à l'unité de calcul (A),
- l'unité de calcul (A) est programmée avec un modèle hydraulique et un modèle cinétique de décroissance du paramètre de qualité considéré,
- et l'unité de calcul (A) met à jour en permanence le modèle hydraulique selon les données de consommation reçues des compteurs (9a, 9b...) pour consommateurs,
- un système de télérelevé fixe (13) qui établit, à partir de toutes les informations de consommation transmises par les compteurs, une estimation de la consommation instantanée, qui est communiquée par une liaison (14) à l'unité de calcul (A), le système de télérelevé (13) étant prévu pour signaler un flux inverse, cette information de flux inverse étant transmise, par une liaison (15), au système de pré-alerte (W).

5. Installation selon la revendication 4, **caractérisée en ce qu'**elle comporte un système de télésurveillance (12) qui reçoit les informations des analyseurs (10a, 10b ...) et qui transmet, après traitement, par une liaison (12a) à l'unité de calcul les informations sur l'état de la pression et du débit dans le réseau, et par au moins une ou plusieurs liaisons (12b, 12c) les informations sur la concentration dans l'eau du paramètre de qualité.

6. Installation selon l'une quelconque des revendications 4 et 5, **caractérisée en ce que** des analyseurs (10a, 10b,...) mesurent la teneur en chlore de l'eau qui constitue un paramètre de qualité.

## Patentansprüche

1. Verfahren zur Überprüfung einer Wasserqualität eines Verteilungsnetzes von Trinkwasser, umfassend einerseits Verbrauchszähler und andererseits auf Überwachungspunkten des Netzes verteilte Online-Analysatoren zur Messung zumindest eines Wassersqualitätsparameters, mit den folgenden Schritten:
- die Messungen der Analysatoren (10a, 10b...) werden an eine Recheneinheit (A) übertragen,
- die Recheneinheit (A) ermittelt geschätzte Werte der Konzentration des betreffenden Parameters im Wasser an den verschiedenen Überwachungspunkten des Netzes,
- die an verschiedenen Punkten des Netzes gemessenen Werte des Qualitätsparameters werden an ein Frühwarnsystem (W) übertragen,
- und das Frühwarnsystem (W) führt einen Vergleich zwischen den geschätzten Werten des Qualitätsparameters und den an verschiedenen Punkten des Netzes gemessenen Werten durch, wobei ein Alarm ausgelöst wird, wenn der Abstand zwischen einem gemessenen Wert und einem geschätzten Wert einen vorbestimmten Schwellenwert überschreitet, **dadurch gekennzeichnet, dass** zur Echtzeitüberprüfung,
- mit Fernerhebungsvorrichtungen ausgestattete Zähler (9a, 9b...) auf Verbraucherabzweigungen installiert sind,
- die Verbrauchswerte der verschiedenen Verbrauchszähler (9a, 9b...) an die Recheneinheit (A) übertragen werden,
- die Recheneinheit (A) mit einem hydraulischen Modell und einem kinetischen Modell des Abfallens des betreffenden Qualitätsparameters programmiert ist,
- und die Recheneinheit (A) ständig das hydraulische Modell entsprechend den von den Verbrauchszählern (9a, 9b...) erhaltenen Verbrauchswerten aktualisiert,
- wobei eine Information über einen Rückfluss ausgehend von einem Zähler (9a,...) an das Frühwarnsystem (W) übertragen wird (15) und die gruppierten Informationen über den Rückfluss und den Gehalt des Qualitätsparameters kombiniert werden, um das Wahrscheinlichkeitsniveau der Erkennung einer Anomalie oder einer Intrusion in das Netz zu erhöhen.

2. Verfahren nach Anspruch 1, **dadurch gekennzeichnet, dass** die Verbrauchswerte der verschiedenen mit Fernerhebungsvorrichtungen ausgestatteten Zähler (9a, 9b...) an die Recheneinheit (A) zumindest einmal pro Stunde übertragen werden.

3. Verfahren nach einem der Ansprüche 1 bis 2, **dadurch gekennzeichnet, dass** der Wassersqualitätsparameter, welcher bei der Echtzeitüberprüfung des Netzes berücksichtigt wird, von einem Chlorgehalt des Wassers gebildet wird.

4. Anlage zur Ausführung eines Verfahrens nach einem der vorhergehenden Ansprüche für ein Verteilungsnetz von Trinkwasser, umfassend einerseits Verbrauchszähler und andererseits auf Überwachungspunkten des Netzes verteilte Online-Analysatoren, zur Messung zumindest eines Wassersqualitätsparameters, wobei die Anlage gleichsam umfasst
- eine Recheneinheit (A), an welche die Messungen der Analysatoren (10a, 10b...) übertragen werden, wobei die Recheneinheit (A) geschätzte Werte der Konzentration des betreffenden Parameters im Wasser an verschiedenen Überwachungspunkten des Netzes erstellt,
- und ein Vorwarnsystem (W), an welches die an verschiedenen Netzpunkten gemessenen Werte des Qualitätsparameters übertragen werden, welches einen Vergleich zwischen den geschätzten Werten des Qualitätsparameters und den an verschiedenen Netzpunkten gemessenen Werten ausführt, wobei ein Alarm ausgelöst wird, wenn der Abstand zwischen einem gemessenen Wert und einem geschätzten Wert einen vorherbestimmten Schwellenwert überschreitet,
**dadurch gekennzeichnet, dass** zur Echtzeitüberprüfung
- die Anlage mit Fernerhebungsvorrichtungen ausgestattete Zähler (9a, 9b...) umfasst, welche auf Verbraucherabzweigungen installiert sind,
- die Verbrauchswerte der verschiedenen Verbrauchszähler (9a, 9b...) an die Recheneinheit (A) übertragen werden,
- die Recheneinheit (A) mit einem hydraulischen Modell und einem kinetischen Modell des Abfallens des betreffenden Qualitätsparameters programmiert ist,
- und die Recheneinheit (A) ständig das hydraulische Modell entsprechend den von den Verbrauchszählern (9a, 9b...) erhaltenen Verbrauchsdaten aktualisiert,
- ein festinstalliertes Fernerhebungssystem (13), welches ausgehend von sämtlichen von den Zählern übertragenen Verbrauchsinformationen eine Schätzung des momentanen Verbrauchs erstellt, welche über eine Verbindung (14) an die Recheneinheit (A) kommuniziert wird, wobei das Fernerhebungssystem (13) vorgesehen ist, um einen Rückfluss anzuzeigen, wobei die Information über den Rückfluss über eine Verbindung (15) an das Frühwarnsystem (W) übertragen wird.

5. Anlage nach Anspruch 4, **dadurch gekennzeichnet, dass** diese ein Fernüberwachungssystem (12) umfasst, welches die Informationen der Analysatoren (10a, 10b...) erhält und welches nach deren Verarbeitung über eine Verbindung (12a) die Informationen über den Zustand des Drucks und des Abflusses im Netz und über zumindest eine oder mehrere Verbindungen (12b, 12c) die Informationen über die Konzentration des Qualitätsparameters im Wasser an die Recheneinheit überträgt.

6. Anlage nach einem beliebigen der Ansprüche 4 und 5, **dadurch gekennzeichnet, dass** die Analysatoren (10a, 10b,...) den Chlorgehalt des Wassers messen, welcher einen Qualitätsparameter bildet.

## Claims

1. A method of monitoring the quality of the water of a drinking water distribution network including on the one hand consumption meters and on the other hand in-line analyzers at surveillance points of the network for measuring at least one quality parameter of the water, in which method:
- measurements from the analyzers (10a, 10b, etc.) are sent to a calculation unit (A),
- the calculation unit (A) establishes estimated values of the level in the water of the parameter concerned at the various surveillance points in the network, and
- measured values of te quality parameter at different surveillance points of the network are transmitted to a pre-alarm system (W),
- and the pre-alarm system (W) compares the estimated values of the quality parameter and the values measured at various points of the network, an alarm being tripped if the difference between a measured value and an estimated value exceeds a predetermined threshold,
**characterized in that** , for monitoring in real time:
- meters (9a, 9b, etc.) equipped with remote meter reading devices are installed on branch connections to consumers,
- consumption data from the various consumer meters (9a, 9b, etc.) is sent to the calculation unit (A),
- the calculation unit (A) is programmed with a hydraulic model and a kinetic model of the decrease of the quality parameter concerned, and
- the calculation unit (A) continuously updates the hydraulic model according to consumption data received from the consumer meters (9a, 9b, etc.),
- an information on reverse flow is transmitted (15) to the pre-alarm system (W) from a meter (9a, etc.) and grouped information on reverse flow and quality parameter level are combined to increase the probability of detecting an abnormality in or an intrusion into the network.

2. The method as claimed in claim 1, **characterized in that** consumption data from the various meters (9a, 9b, etc.) equipped with remote meter reading devices is sent to the calculation unit (A) at least once per hour.

3. The method as claimed in any of claims 1 to 2, **characterized in that** the water quality parameter taken into account for real time monitoring of the network is the chlorine concentration of the water.

4. An installation for implementing a method as claimed in any of the preceding claims for a drinking water distribution network that includes on one hand consumption meters and on the other hand in-line analyzers at monitoring points in the network to measure at least one water quality parameter, which installation also includes:
- a calculation unit (A) to which is sent measurements from the analyzers (10a, 10b, etc.) and said calculation unit (A) establishing estimated values of the level in the water of the parameter concerned at the various surveillance points in the network,
- and a pre-alarm system (W) to which are transmitted the measured values of the quality parameter at various points in the network and that compares the estimated values of the quality parameter and the values measured at various points in the network, an alarm being tripped if the difference between a measured value and an estimated value exceeds a predetermined threshold,
**characterized in that** , for monitoring in real time:
- the installation includes meters (9a, 9b, etc.) equipped with remote meter reading devices installed on branch connections to consumers,
- consumption data from the various consumer meters (9a, 9b, etc.) is sent to the calculation unit (A),
- the calculation unit (A) is programmed with a hydraulic model and a kinetic model of the decrease of the quality parameter concerned, and
- the calculation unit (A) continuously updates the hydraulic model according to consumption data received from the consumer meters (9a, 9b, etc.).
- a fixed remote meter reading system (13) which establishes from all the consumption information sent by the meters an estimated instantaneous consumption that is communicated via a link (14) to the calculation unit (A), the remote meter reading system (13) being adapted to signal a reverse flow, said reverse flow information being sent over a link (15) to the pre-alarm system (W).

5. The installation as claimed in claim 4, **characterized in that** it includes a remote surveillance system (12) which receives information from the analyzers (10a, 10b, etc.) and which sends to the calculation unit via a link (12a), after processing it, information on the pressure and flow rate state of the network and via one or more links (12b, 12c) information on the level of the quality parameter in the water.

6. The installation as claimed in any of claims 4 and 5, **characterized in that** analyzers (10a, 10b, etc.) measure the chlorine concentration of the water, which constitutes one quality parameter.
